# EUROPEAN PATENT APPLICATION

(11) **EP 2 486 858 A1**
(43) Date of publication of application: **15.08.2012**
(21) Application number: 12152141.3
(22) Date of filing: 23.01.2012
(51) Int. Cl.: A61B 17/064, A61B 17/70

(54) **Method of making an elongated wire for an orthopaedics implant**

(30) Priority: 09.02.2011 US 201113024479
(71) Applicant: Groiso, Jorge Abel, Buenos Aires (AR)
(72) Inventor: Groiso, Jorge Abel, Buenos Aires (AR)
(74) Representative: Monzón de la Flor, Luis Miguel

(57) **Abstract**

A device for correction of bone and/or soft tissue disorders and methods for obtaining the device, the device comprising an elongated wire entirely made of a shape-memory alloy and thermally treated and mechanically deformed to have at least one portion exhibiting a shape memory characteristic and a desired shape, and at least one remaining portion without exhibiting said shape memory characteristic.

## Description

### BACKGROUND OF THE INVENTION

### FIELD OF THE INVENTION

The present invention relates to devices, tools and means for application in the medical field and particularly to orthopaedics and traumatology for correcting bone deformities, disorders and deficiencies. More particularly, the invention relates to a device such as a clip, implant or staple and method for obtaining the same, wherein the device is employed in correcting bone deficiencies such as abnormal curvatures by suppressing and/or inducing the growth of the bone mass or stretching of soft tissues, and/or the epiphyseal plate in the bone, correcting abnormally curved spinal columns, distracting or contracting the vertebrae at the concave and/or convex side of a deformed column, or diseases of the adult spine as spinal stenosis or disk disturbances.

While more specific reference to disorders in the spinal column will be made in the present disclosure, the bone implant and methods of the invention are not restricted to this specific application but they may be applied to any other field where a prosthesis, a fixation device, a correction device, etc. is to be secured into a bone, proximal bones or a broken bone for correction and/or reparation purposes. In addition, the term "patient" is applicable either to animals or human beings.

### DESCRIPTION OF THE PRIOR ART

Length discrepancies and angular deformities of the limbs and spine are quite frequent among children and adults and provoked by several causes such as congenital, post-trauma, post-infection, oncologic causes, etc. The need exists for correcting these abnormalities because of function and aesthetic reasons. One of the treatments of some of these childhood deformities is based on the concept of mechanically suppressing growth.

Bone staples are well known in the orthopaedics and traumatology, such as in techniques for correcting angular deviations of limbs, genu valgum for example, as well as spine or column deviations, by unilateral restriction of the growth plate.

For correcting angular deviations the mechanical suppression can be obtained by temporary using metal staples across the growth plate, at unilateral locations, at the convex side of the deformity, for example. The staples are removed once the correction has been reached.

For correcting length abnormalities, the lengthening of the bone is obtained by using an external fixator and the shortening of the limb is obtained by arresting or suppressing the growth plate at even locations around the bone.

The technique of mechanical distraction by temporary using an external fixator is carried out by installing the fixator in the limb, for producing a slow stretching of the growth plate. This mechanical technique is known as epiphyseal distraction.

More precisely, epiphyseal distraction is a surgical technique that is used to lengthen an abnormal bone or correct physeal deviations prior to growth plate closure. This technique, also called "chondrodiatasis", involves slow, gradual, symmetric distraction of the growth plate. The term "hemichondrodiatasis", on the other hand, is used to refer to the technique of asymmetric distraction of the growth plate for correction of epiphysealdiaphyseal deviations.

Since the use of external fixators has shown to be expensive as well as traumatic and cumbersome for the patient, and the fixator leaves unsightly scars, the use of this device has not widely and intensively imposed. These fixators also can damage the growth plate.

Many known clips, staples and other devices are preferably made of nitinol (NiTi), a biocompatible, shape memory metal alloy of titanium and nickel. A shape memory material can assume an initial high temperature configuration and a deformed low temperature configuration and revert back to the initial configuration upon the application of heat. Thus, the staples have the capability of being bent when cooled and reform to their original shape when reheated. The metal changes shape with temperature or under the influence of stress because of crystalline phase changes. Specifically, the metal transitions from an austenitic state to a martensitic state with a decrease in temperature. According to one of the ways to implant a staple, the SMA staple is cooled and then deformed while at a temperature less than the transformation temperature at which it is in the martensitic phase. The staple is then inserted in its deformed shape and when heated, under the body temperature, will reform to its original shape. While it has been desirable to use staples for the above mentioned techniques, the staples are affected by several disadvantages and drawbacks that caused them to be not widely imposed in the medical field.

As it was stated above, angular deviations or deformities and length discrepancies of the limbs, for example, are often treated with staples. After staples are placed across a growth plate, further growth of this plate increases the pressure on the inner surfaces of the staple legs. This increased pressure, which has been measured about 1 Mpa, slows bone growth within and near the staple, i.e. in the region between the legs, and, simultaneously, forces the staple legs apart. This pressure from the cartilage plate, together with the legs moving away from each other, causes the staple legs to be extruded and expelled out from the bone thus loosing the grip in the bone.

In effect, the pressure exerted by the growing cartilage plate causes the staple legs to open, extrude and slide out of the bone. Many staples of the prior art has attempted to prevent the staple from sliding out the bone by providing the staple legs with barbs or protrusions to enhance the anchoring of the legs into the bone mass. However, when the deformation has been corrected and the temporary staple must be removed from the bone, the anchorage of the legs into the growing bone mass, desired at the time the stable must be retained into the bone, is a bar or obstacle at the time the staple must be removed. In effect, this firm osseous anchorage prevents the staple from being easily removed by normal forces and operations.

The above drawback is also found in the application of the staples in bone malformations or disorders with the purpose of correction of abnormal curvatures in spinal columns, such as scoliosis. There are several devices, systems, implants and staples for connecting to the vertebrae to exert a force or combination of forces to urge the column and bring the same as close as possible into its normal curvature.

The distraction of soft tissues has also been used for therapeutic purposes: skeletal traction in general, the use of an external fixator to relieve the intraarticular pressure on diseased joints, interdigital cutaneous stretching for syndactilia, correction of retractile scars. It also has been found that vertebral distraction is also helpful for the treatment of some spine disorders: disk herniations, spinal stenosis.

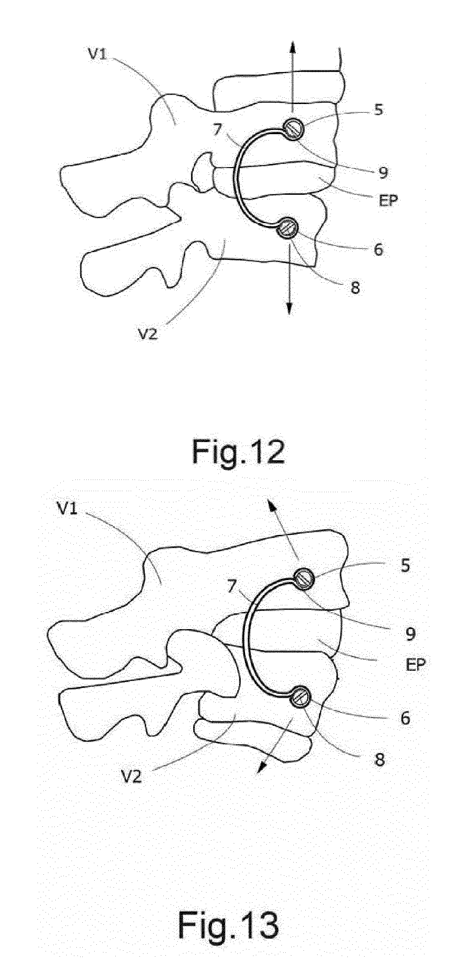

In order to firmly affix the staple in the bone, some staples are provided with holes at the ends of the staple in order to receive screws to be screwed and retained into the bone mass, See U.S. Patent No. 7,297,146 discloses an implant for stabilizing a bony segment, with an embodiment, as shown in FIGS. 13-14, being a distraction implant 700 including a body 702 extending from a first end 704 to an opposite second end 706. Ends 704, 706 including respective holes 710, 712 to facilitate intravertebral attachment to a vertebral body with, for example, a first anchor 21 and a second anchor 30. Ends 704, 706 can each be enlarged relative to body 702 to form the respective holes 710, 712 to receive an anchor therethrough. Distraction implant 700 can be formed of a shape-memory material (shape memory alloy "SMA") that exhibits superelastic characteristics or behaviour at about human body temperature but it is disclosed that only a portion between ends 704, 706 may be at least partially formed of the shape-memory material, with the ends 704, 706 formed of any suitable biocompatible material, such as, for example, stainless steel or titanium. Therefore, to take advantage of the "SMA" only at the portion between ends of the staple, two materials are employed, the "SMA" for the intermediate portion and stainless steel, for example, at the ends. This combination of two different materials may be inconvenient for the behaviour of the implant because any attempt to join or weld the different materials demands the heating of the materials to a temperature very close or at the melting point, the metals could transform it selves by several mechanisms, (oxidation, precipitation, crystallisation, etc.) and lose their biocompatibility.

US Patent No. 6,325,805 discloses a vertebral interbody staple 100 having generally U-shape with crossbar 101 between legs 102 and 103 with leg 102 having a pointed tip 104 and leg 103 having a pointed tip 105 for insertion into the vertebral bodies. Leg 102 has barbs 106, 107 and leg 103 has barbs 108, 109 for prevention of staple backout. The staples are made of nitinol, a biocompatible, shape memory metal alloy of titanium and nickel. Staples are capable of being bent when cooled and reform to their original shape when reheated. In one embodiment the SMA staple is cooled and then deformed while at a temperature less than the transformation temperature at which it is in the martensitic phase. The staple is then implanted and, at the corporal temperature, returns to its original shape. This document also discloses that at least a portion of the staple is manufactured from a shape memory material. If this is so, it is understood that a portion is made of "SMA" while the remaining portion of the staple is made of another different material.

In both above US Patents the possibility of having a staple made of two different materials is disclosed, to have the benefit of the different behaviour or characteristics of both materials, such as a portion with shape memory and another portion with characteristics not affected by the alteration of the temperature at normal rates. However, as explained above, this is very inconvenient for medical devices, particularly for implants that must remain stable, must keep uniformity and no metal interfaces should be permitted to keep a material integrity to guarantee resistance to stresses, fatigue, etc.

As indicated above, Nitiniol made medical devices are well known in the art. This shape memory alloy transitions from an austenitic state to a martensitic state with a decrease in temperature. As the alloy transitions from the austenitic state to the martensitic state it becomes more easily deformed. In the martensitic state, the alloy is able to accommodate significant plastic deformation at an almost constant stress level. Upon heating a metal in the martensitic state, the metal begins to return to an austenitic state and reverts to its initial configuration.

It is also well known that by selective local heat treatment it is possible to destroy the austenitic-martensitic transition and/or change the elasticity or stiffness of a shape memory material. Based on this, US 6.485.507; US 6,540,849; 6,106,642 and 6,997,947 to Walak et al. disclose a Nitinol made device locally treated by heat to change the properties of the material, such as elasticity or stiffness, in a manufactured device. Walak et al, disclose to treat a medical device such as a stent and a guide wire to alter the properties of the device just in a restricted portion thereof. To do this, Walak performs a first annealing and a subsequent second annealing at different times and temperatures. As disclosed in US 6,485,507, Column 6, lines 45-67, Walak indicates that the purpose of heat treating a region 208 of a guide wire is to have the region with tendency towards plastic deformation of the treated portion relative to the remainder of the guide wire, therefore if the remainder is forced to deformation, during use of the device, the part having the shape-memory property comes back to the original shape while region 208 remains deformed. Walak proposes to have a shape memory material made device to have a portion thereof capable of being plastically deformed when the entire device is forced to have a momentary deformation of the shape memory portion during use. Specifically, a region of a shape memory material is treated to decrease the stiffness, and increase ductility, of the region relative to the remainder of the device. Walak does not seek for treating a portion of the device to deform the same into a design capable of being maintained firm and rigid enough to prevent any deformation thereof when the remainder of the medical device is deformed in the martensitic phase and when it recovers the original shape in the austenitic phase.

It would be therefore very convenient to have a new staple having at least two different characteristics, such as the ones of a "SMA" and the ones of a stainless steel, for example, but without the need of joining two different materials but having such characteristics in a piece integrally made of a "SMA" material, with at least a portion of the piece being thermally treated to eliminate, cancel out or remove the shape memory and super elastic characteristics or "SMA" effect and being mechanically treated to deform the thermally treated portion into a desired shape capable of being permanently maintained.

### SUMMARY OF THE INVENTION

It is therefore an object of the invention to provide a device for joining at least two bone pieces or to distract a bone and/or a soft tissue, having an intermediate portion to exert a force in the bone or bone pieces and at least one end having retention means to affix the device in the bone, with the intermediate portion having a shape memory characteristic.

It is still another object of the present invention to provide a new staple having at least two different characteristics, such as the ones of a "SMA" and the ones of a common metal, such as a stainless steel, for example, but without the need of joining two different materials but having such characteristics in a piece integrally made of a "SMA" material, with at least a portion of the piece being thermally treated to eliminate, cancel out or remove the shape memory characteristics.

It is a further object of the present invention to provide a device for correction of bone and/or soft tissue disorders comprising an elongated wire entirely made of a shape-memory alloy and thermomechanically treated to have at least one portion exhibiting a shape memory characteristic and at least one remaining portion without exhibiting said shape memory characteristic, and with the at least one remaining portion mechanically deformed during the thermal treating thereof to have a permanent desired shape.

It is a further object of the present invention to provide a distraction/contraction implant made of a shape memory alloy and having at least a portion thereof thermally treated to eliminate the shape memory characteristics of the alloy without the characteristics of the remaining portion being altered, with the at least one portion being also deformed into a desired shape during the thermal treatment.

It is a further object of the present invention to provide a staple for correction of soft tissue or bone disorders comprising:
an elongated wire entirely made of a material exhibiting a shape memory characteristic, the elongated wire having two opposite ends and an intermediate portion extending between the two opposite ends,
wherein at least one of the two opposite ends is thermally and mechanically treated in a manner to deform and eliminate the shape memory characteristic of the elongated wire just in said at least one opposite end while keeping the shape memory effect in the intermediate portion of the wire.

It is a further object of the present invention to provide a method of making a staple comprising:
an elongated wire entirely made of a material exhibiting a shape memory characteristic, the elongated wire having two opposite ends and an intermediate portion extending between the two opposite ends,
wherein at least one of the two opposite ends is thermally and mechanically treated in a manner to eliminate the shape memory characteristic of the elongated wire just in said at least one opposite end while keeping the shape memory effect in the intermediate portion of the wire,
the method comprising the steps of:
providing a length of a wire made of a material exhibiting a shape memory characteristic,
defining two opposite ends and the intermediate portion of the wire, with the intermediate portion extending between the two opposite ends,
thermally and mechanically treating at least one of the two opposite ends to eliminate the shape memory characteristic of the elongated wire and deformed into a desired shape just in said at least one opposite end while keeping the shape memory effect in the intermediate portion of the wire.

It is a further object of the present invention to provide a device for correction of bone disorders comprising a body made of a material exhibiting a shape memory characteristic, the body having a first portion thermally treated and mechanically deformed in a manner to have the shape memory characteristic eliminated and a desired shape, and a second portion exhibiting said shape memory characteristics.

It is a further object of the present invention to provide a method of making a device for correction of bone disorders comprising an elongated wire entirely made of a shape-memory alloy and thermally treated to have at least one portion exhibiting a shape memory characteristic and at least one remaining portion without exhibiting said shape memory characteristic, with the method comprising the steps of:
providing a length of a wire made of a shape memory alloy;
defining at least one portion and at least one remaining portion of the wire;
forming the at least one portion of the wire into a desired form;
thermally treating the at least one remaining portion of the wire in order to set the at least one remaining portion to exhibit a shape memory characteristic.

It is another object of the present invention to provide a method of making a device comprising a body made of a material exhibiting a shape memory characteristic, the body having a first portion thermally treated and mechanically deformed in a manner to eliminate the shape memory characteristic and have a desired shape, and a second portion exhibiting said shape memory characteristics, the method comprising:
providing a body made of a material exhibiting a shape memory characteristic,
defining a first portion and a second portion of the body,
thermally treating the first portion of the body to eliminate the shape memory characteristic of body while keeping the shape memory effect in the second portion of the body, and mechanically deforming, during the thermal treating, the first portion to bring it into a desired shape.

The above and other objects, features and advantages of this invention will be better understood when taken in connection with the accompanying drawings and description.

### BRIEF DESCRIPTION OF THE DRAWINGS

The present invention is illustrated by way of example in the following drawings wherein:
Figure 1 shows a Nitinol wire before treated by means of the method according to the present invention;
Figure 2 shows a staple with their ends already thermally treated and formed into rings according to the teachings of the present invention and having the memory shape at a room temperature;
Figure 3 shows the staple of Figure 2 deformed under the Austenite Temperature Ap, generally at a cool temperature, at which the device may be easily deformed as it behaves as a simple plastic material;
Figure 4 shows a perspective view of the staple of Figure 2 with corresponding screws arranged at the ring shaped ends of the staple;
Figure 5 shows a side elevation view of a staple implanted in a bone, across the epiphyseal plate, to promote controlled deformation in the bone, according to the invention;
Figure 6 shows a front elevation view of the staple implanted in a bone, according to Figure 5;
Figure 7 shows a side elevation view of a staple implanted in a bone, across the epiphyseal plate, before a controlled deformation in the bone, according to the invention;
Figures 8A, 8B show respective side elevation views of the staple of Figure 7, with the bone controllably deformed in the shaded areas, a triangular one above the epiphyseal plate, Fig. 8A, and a peripheral one at the bottom end of the bone, all according to the invention;
Figure 9 shows a partial cross-sectional side elevation view of an end ring of a staple of the invention with a screw mounted therein and at the situation illustrated in Figure 7, before a controlled deformation in the bone;
Figure 10 shows a partial cross-sectional side elevation view of an end ring of a staple of the invention with a screw mounted therein and at the situation illustrated in Figures 8A, 8B, with the ring and the screw being the one shown at the bottom of Figures 8A, 8B, after a controlled deformation in the bone;
Figure 11 shows a partial cross-sectional side elevation view of an end ring of a staple of the invention with a screw mounted therein, together with a nut and washer to prevent pivoting of the screw relative to the ring end of the staple;
Figure 12 is a side elevation view of a spinal column having a device according to the invention, in its deformed status or shape, implanted in two proximal vertebrae;
FIG. 13 is a side elevation view of the device and spinal column of Figure 12 as it is after a period of treatment time, with the device in its memorized status or shape, and
FIG. 14 is a posterior elevation view of a spinal column having a device according to the invention, in its deformed status or shape, connected to two proximal vertebrae by screws.

### DESCRIPTION OF THE PREFERRED EMBODIMENTS

Now referring in detail to the invention, the same refers to a new medical device made of an integral piece of a shape memory material such as an alloy, preferably NiTi (nitinol), with the piece thermally treated to have differentiated physical and mechanical properties along predetermined parts of the piece.

A Nitinol shape memory or superelastic element generally requires the setting of a desired shape for a particular application and this involves a process comprising constraining the Nitinol element on a mandrel or fixture of the desired shape and applying an appropriate heat treatment. The high temperature configuration of the material is set into the material or memorized during an initial shaping step in which the material is maintained at a high temperature in a desired shape for a period of time. This process is carried out to all forms of Nitinol, either in the form of wire, ribbon, strip, sheet, tubing, or bar.

The heat treatment, carried out by the manufacturer to set the form of the piece, generally employs closer to 500 degrees C and times over 5 minutes and the cooling is rapid preferably via a water quench or rapid air cool. A shape memory alloy, a Nitinol piece, as provided by the manufacturer is already set at a typical Austenite Final Temperature or Active Temperature Af at which the device recovers its memory shape as set by the manufacturer. The device is also set at a typical Austenite Peak Temperature Ap at which the device may be easily deformed as it behaves as a simple plastic material. For orthopaedic devices, for example, the temperature Ap is set in a range of -15°C to 15°C and the Af temperature is set at a range of 10°C to 40°C, generally the body temperature.

According to the invention, a piece of nitinol, preferably an elongated member and more preferably a nitinol member in the form of a wire, is taken as provided by the manufacturer and mechanically and thermally treated in at least one portion thereof, preferably in an end thereof, and most preferably in both ends of the piece, to eliminate the shape memory characteristics, with the provision that the remaining portion of the piece out of that at least one portion is kept unaltered, that is with the remaining portion maintaining the shape memory characteristics as set by the manufacturer. The term "eliminates" means also to almost eliminate, to cancel out or to remove the shape memory characteristics or "SMA" effects.

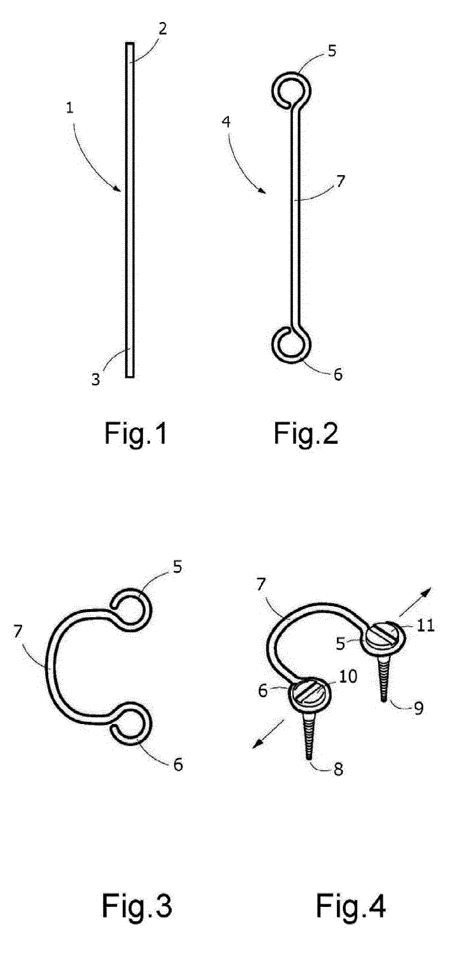

The inventive device as shown in Figures 2-4, is formed by selectively thermomecanically treating a NiTi-made piece, preferably a piece on NiTi wire indicated by 1 in Figure 1. The piece may correspond to any NiTi piece commercially available from any desired manufacturer and preferably a straight wire having a proper diameter, oxide free, Nickel 55.66%. Preferably, the chemical composition of the alloy is as follows:

| | |
|---|---|
| Nickel | 55.77% |
| Titanium | balance |
| Oxygen | 0.05 maximum |
| Carbon | 05 maximum |
| Si, Cr, Co, Mo, W, V | < 0.01 |
| Nb, Al, Zr, P, Cu, Ta, Hf | < 0.01 |
| Ag, Pb, Bo, Ca, Mg, Sn, Cd | < 0.01 |
| Zn, Sb, Sr, Na, As, Be, Ba | < 0.01 |
| Fe | < 0.05 |
| B | < 0.001 |

Wire 1 may have any necessary diameter, depending of the use and application, and has a pair of ends 2, 3 and, according to the invention, is thermally treated and formed into the shape depicted in Figure 2 to define a medical device, such as for example an implant device, now indicated by reference 4, with at least one of the two opposite ends 2, 3, and preferably both ends 2, 3, being thermally treated in a manner to eliminate the shape memory characteristic of the elongated wire just in said at least one end or both opposite ends while keeping the shape memory effect in the intermediate portion of the wire. More particularly, end 2 and/or end 3 are heated up to a temperature enough to make them malleable to be formed, for example mechanically deformed, into a desired shape, such as a ring 5, 6, and an intermediate portion 7. Only a short length of ends 2, 3 are thermally treated necessary to form rings 5, 6, while intermediate portion 7 is prevented from being heated during the treatment of the ends.

Having knowledge about the benefits of the "SMAs", the inventor has been seeking for a new staple having the shape memory characteristics of a "SMA" to exert a compression or distraction constant forced in a bone or a soft tissue, with the addition of means for retaining the staple in the bone. These means should be capable of receiving screws to be screwed into the bone and must be capable of remaining rigid to receive such screws and to permit screwing thereof into the bone. Since a NiTi staple is currently cooled at a temperature at which the material becomes soft and malleable in order to manipulate the same to easy installation into the patient's body, if rings 5, 6 are formed in ends 2, 3, of wire 1 conserving its shape memory characteristics, rings 5, 6 would be soft and malleable like the rest of the wire and no support for screws would be provided at the time of insertion of the staple into the bone. Thus, the inventor was seeking to have rings 5, 6 not only without "SMA" characteristics but with a stiffness enough to work as retaining means. Seeking for this property, the inventor not only heated the desired ends but also, when heated, mechanically deformed the ends into retaining means, such as rings 5, 6, with the rings resulting firm and rigid to retain screws 8, 9 without being plastically deformed under the stresses generated by intermediate portion exhibiting the shape memory properties and recovering the original shape under the body's temperature. Distinct from Walak, that proposes to selectively heat treat a region of the device to have a reduced stiffness to permit further deformation of the region when subject to a level of strain, Patent '507 to Walak, col. 5, lines 43-46, col. 6, lines 64-67, etc., the present invention proposes both heat treating a desired region and deforming the same into a desired shape to make the region firm, stiff or rigid. When referring to firm, stiff and rigid it is the purpose of this specification to mean that the heat treated region of this invention must be firm or rigid enough to retain screws 8, 9 without being deformed under the strain imposed by the remainder of the device as it recovers its original shape.

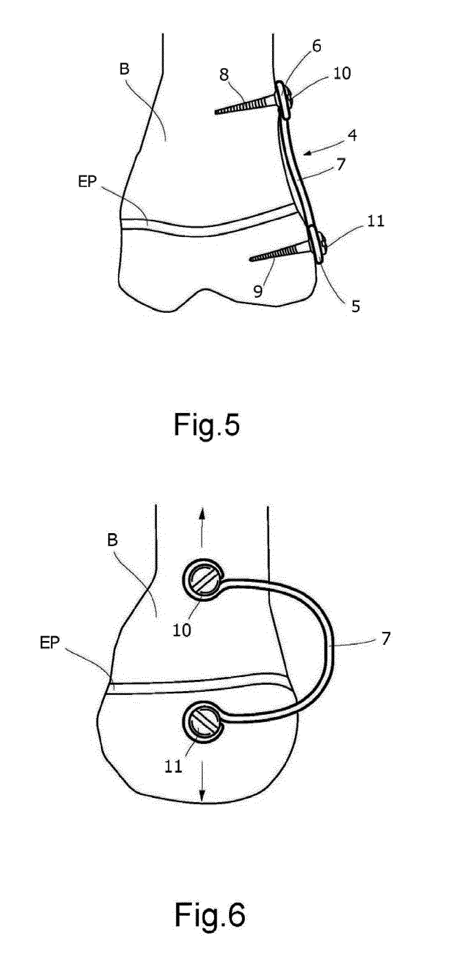

With portion 7 having the "SMA" effect and rings 5, 6 lacking this effect, device 4 may be affixed to a bone "B" under treatment as shown in Figures 5, 6, for example. Thus, device 4 extends across the growth plate "EP", and fixed by screws 8, 9 in rings 5, 6, in order to promote a local growing of the bone mass. However, device 4 may be screwed in other parts of the bone, out of the growth plate also to promote a controlled deformation of the bone, stretching of soft tissues, or to retain two broken parts of the bone together to promote osseous joining. If necessary, device 4 may be comprised of more than one wire 1, for example two or more twisted wires.

According to one alternative of the invention, the piece ofNiTi, such as the wire 1, may be provided without the thermal treatment carried out by the manufacturer to set the desired shape which the piece will have or recover at the temperature of the patient's body. In this alternative, at least one end, such as ends 2, 3, may be formed into a desired shape, such as rings 5, 6, by means of any desired technique, either a thermal technique or a mechanical technique. Once the ends are formed into the desired form, at least one remaining portion of the shape memory alloy body may be thermally treated to set the at least one remaining portion to exhibit a shape memory characteristic, preventing the ends from being set to have such a characteristic.

Thus, according to another aspect of the invention, a method is also provided to make the inventive device and comprising the steps of providing a length of a wire made of a shape memory alloy; defining at least one portion and at least one remaining portion of the wire; forming the at least one portion of the wire into a desired form; and thermally treating the at least one remaining portion of the wire in order to set the at least one remaining portion to exhibit a shape memory characteristic. As indicated above, the temperatures and times to thermally treat the Shape Memory Alloy, such as NiTi, to set the same to have shape memory characteristics and super elasticity, are well known in the art.

According to another aspect of the invention, a method of making the inventive staple 4 is also provided. The method comprises the steps of:
providing a length of a wire 1 made of a material exhibiting a shape memory characteristic,
defining two opposite ends 2, 3 and an intermediate portion 7 of the wire, with the intermediate portion extending between the two opposite ends,
thermally treating at least one of the two opposite ends, 2 or 3 or both, to eliminate the shape memory characteristic of the elongated wire just in said at least one opposite end while keeping the shape memory effect in the intermediate portion 7 of the wire.

The inventor thus heated a portion of wire or a short length of each end 2, 3 up to a temperature at which the ends were able to be easily mechanically deformed by means of a little and conventional tool, such a pliers or pincers. Preferably, the temperature was between about 500°C and well below 1300°C, preferably between about 600° and about 800°, most preferably between about 600° and about 700°, and at this temperature, the ends were mechanically formed into respective circular shapes or rings 5, 6 and, thus deformed, the rings were left to cool down at room temperature. In one example, the ends were heated up to the ends glow red. In other words, the ends were subject to an annealing treatment and mechanically formed into rings 5, 6. In this way, rings 5, 6 loosed their shape memory characteristics while intermediate portion 7 kept this characteristics unaltered. In accordance to the invention, the treated region results with no tendency to plastic deformation. On the contrary, the treated region is firm and rigid enough to resist the stress exerted by the intermediate portion under the shape memory effect at the body's temperature. If the ends of the wire would be treated as disclosed by Walak, screws 8, 9 would deform the ring ends under the stress of the intermediate portion with the screws resulted removed out of the rings. More particularly, the method of the invention is carried out not only to thermally treat a portion of the wire just to change the shape memory properties or the superelastic properties but also to mechanically deform the thermally treated portion into a desired shape different from the original shape of the wire and stiff enough to work as retaining means.

The heating of the ends of the wire were carried out in several ways. One was to heat the ends in a gas burner with heat being concentrated into the ends only. To improve the isolation of intermediate part 7, this portion was also covered with an insulating fabric or tape. According to another alternative, the ends of the wire were inserted into respective holes in the wall of a furnace to heat the ends only while the remaining part of the wire remained out of the furnace. The furnace may be provided with inert gas to prevent oxidation of the heated ends. An insulating tape was also employed alternatively. Still another alternative is heating the desired portion by induction, by using a coil for example, or by a hot inert gas.

Since the melting point of NiTi is 1300°C, the annealing according to the invention should be carried out a temperature below 1300°C. Since precipitation of Nickel may occur, a surface barrier layer such as titanium oxide, titanium nitride, or titanium carbide has been shown to increase the corrosion resistance and biocompatibility, see "Shape memory alloys Manufacture, properties and applications", ed. H. R. Cheng 2010. ISBN:978-1-60741-789-7, Nova Science Publishers, INC, New York, 2010.

Without attempting to support this by a theory, it may be said that upon annealing, partial or complete annealing, the treated portion of NiTi apparently was still austenitic but it was not longer exhibiting any shape memory or superelastic properties or at least these characteristics reduced as a result of the structure of the material may be going through a phase change. With slow cooling down at room temperature the material is going through several solid phases (crystalline) also named "stable phases" that, under the annealing may have precipitated thus changing the material characteristics, se "Shape Memory Materials" Ed. K. Otsuka and C.M. Wayman. Cambridge University Press, UK, 1998.

According to the invention, the piece was heated and let cool down at room temperature, which is different to a quenching treatment wherein the heated material should be cooled down rapidly, generally by immersion in cold water or oil. In order to reduce any brightness or fragility that may result from the annealing, the heat treatment may be combined with cold working as it is well known in the art.

Now, in order to implant staple 4 according to the invention, the staple is first cooled at a temperature of about -5°C to about 5°C in order to easily deform the staple as shown in Figure 3. Thus deformed, screws 8, 9 are inserted into rings 5, 6 in a manner that heads 10, 11 of screws 8, 9 remain retained in the rings, and preferably pivotally retained therein. Wire 1 will preferably have a circular cross-section, whereby screws 8, 9 will be retained with capacity to pivot into rings 5, 6. However, wire 1 may be provided with other cross-sections such as rectangular, square or polygonal cross sections.

With staple 4 at a cool temperature and affixed to a bone "B" extended over and across the epiphyseal plate "EP", the staple will be heated by the body's temperature and will try to recover its original shape as illustrated in Figure 2. Thus, the staple, as deformed in Figures 3-6 will exert a constant diverging force as illustrated by the diverting arrows in Figures 4 and 6.

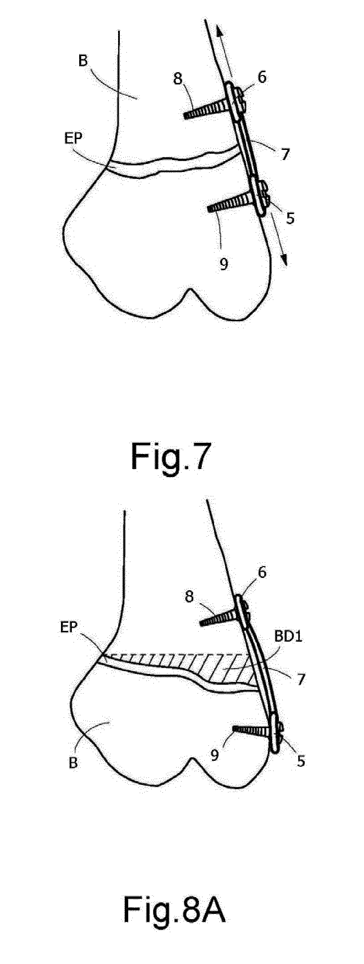

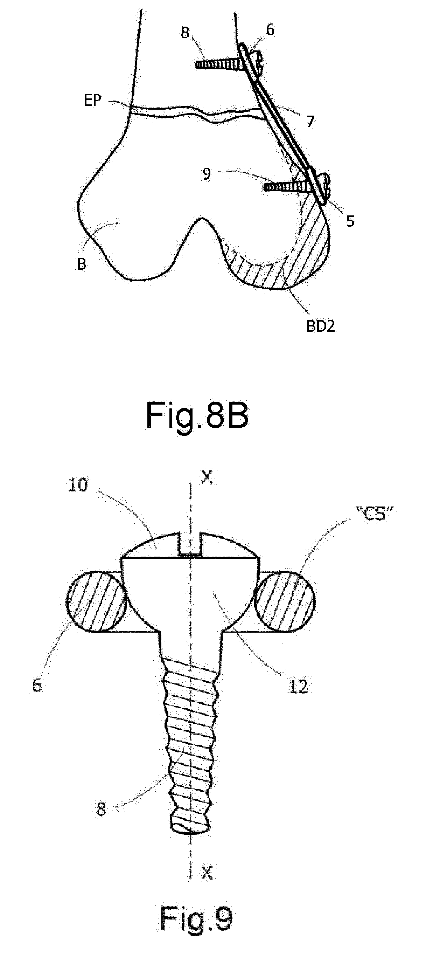

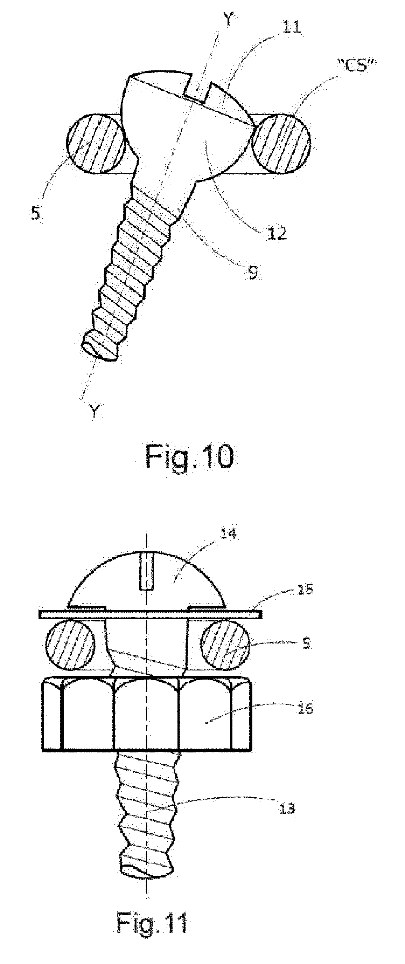

As mentioned when describing Figures 5, 6, the distraction force constantly and continuously exerted by staple 4 will promote the growth of the bone at a desired controlled pattern. For example, as shown in Figures 7, 8A, 8B, staple 4 is implanted extended across the epiphyseal plate "EP" and fixed by screws 8, 9 to the bone "B" with the device being at a low temperature, about -5°C to about 5°C, for example, to be easily deformable. When subject to the temperature of the body, staple 4 will try to recover its original shape exerting the force illustrated by the arrows in Fig. 7. After a period of time the bone will be deformed as shown in Fig. 8A or as shown in Fig.8B. One of the expected deformations is the one indicated by "BD1" in Fig. 8A, with the bone grown from the epiphyseal plate EP, and another deformation that may be expected is that one occurring at the periphery on the bottom end of the bone, as indicated by "BD2" in Fig. 8B. To promote this controlled bone formation, according to the invention, circular cross section "CS" of staple 4, allows pivotal movement of the screws, as shown in Figs.9, 10. Fig. 9 shows a partial cross sectional view of screw 8 of Figures 8A, 8B, that remains in the same position after bone deformation, however, screw 9 was able to pivot relative to ring 5, as illustrated in Figures 8A, 8B, 10, to accommodate to the bone formation, thus permitting to exert an asymmetric pressure to obtain an unilateral deformation of the bone. In a test on calves and lambs, the constant distraction exerted by an implant staple of the invention a desired bone deformation was reached at two months from implantation. With this new arrangement between the rings and screws the use of pedicle screws are no longer necessary. The screws to be employed with the inventive staple may be of simple type such as the ones illustrated in Figs. 9, 10, preferably with a bottom 12 of screw head 10, 11 being rounded.

The arrangement of Figs. 7-10 have been designed to permit pivotal movement between the screw and the staples, however, it may be convenient to prevent this movement if a bone deformation is seeking wherein the screws must keep a constant relative position. In this event, the arrangement depicted as a partial cross section in Fig. 11 is provided also according to the invention. A screw 13 having a head 14 preferably with a flat bottom, is inserted into ring 5 of staple 4 provided with a washer 15 and a nut 16, in a manner that ring 5 is sandwiched between washer 15 and nut 16 as shown. This will guarantee a firm and constant relative position, preferably perpendicular, between ring 5 and screw 13. Thus, the distraction force of staple 4 will be transmitted to the bone not only at the region of the screw head, as in Figures 8A, 8B, but all along the length of the screw.

According to even a further aspect of the invention, the inventive device is provided to correct a spinal column that is curved out of its natural curvature. As shown in Figs. 12, 13, staple 4 is implanted or affixed to two adjacent vertebrae "V1", "V2", extending across and above the epiphyseal plate "EP". The purpose of implanting staple 4 is to promote separation of the vertebrae that are inconveniently close to each other, as shown in Fig. 12, to bring them to a position as shown in Fig. 13. See Lee J, Hida K, Seki T, et al. An interspinous process distractor (X STOP) for lumbar spinal stenosis in elderly patients: preliminary experiences in 10 consecutive cases. J Spinal Disord Tech.2004; 17(1):72-7. See also Kim Y.Z., Zhang H.Y., Moon B.J., Park K.W., Ji K.Y., Lee W.C., Oh K.S., Riu G.U. Kim D.H. Authors of "Nitinol spring and dynamic stabilization system and Nitinol memory loops in surgical treatment for lumbar disc disorders: short-term follow up," published in Neurosurgical Focus, vol. 22, no. 1, January 15, 2007. It is also useful to relieve the force of gravity, when a curve is present, the gravity worsen the deformity. As already explained above, the staple is thermally treated to settle a temperature range, for example, from about 35° to about 45°, at which temperature the shape of the staple is memorized to be straight as shown in Fig. 1, to exert a distraction force when deformed out of its memorized shape.

The implant is brought to a deformed shape, as shown in Fig. 12, in order to connect the same to the walls of two proximal vertebrae. The implant may be deformed into the shape shown in Fig. 12 by cooling the implant at a temperature preferably between about -5° and about 5°C. The heating of the staple to a temperature between about 35° and about 45°, that is a temperature range including a normal temperature of the spinal column, may be made by the simple contact of the staple with the corporal temperature or by a heated saline solution, or by using low voltage electrical current, direct or percutaneous ultrasonic waves, and general high frequency waves concentrated into the staple.

Epiphyseal plate "EP" is the place where longitudinal growth of the spine takes place. Plate "EP" has a circular ring that contributes to the increase in the size of the vertebral body and the end plate is similar to the physis of the long bones, therefore, for the purposes of this description, end plate and epiphyseal plate will be used indistinctly.

In connection to curvature abnormalities, during the development of a scoliotic curve, while the body of the vertebrae remains with the normal shape, the deformity occurs at the intervertebral spaces. The Nitinol divergent staple of the invention allows the application of a traction force to the intervertebral space and particularly to the end plate of a vertebra. The proposed treatment would work on the intervertebral space and the end plates. The staple distracts the space where the tissues are being compressed, for instance a concave side of a deformed spine. It also could be used among adults and older people, for disk disorders or spinal stenosis, where the vertebral distraction has been shown to be very useful as a treatment.

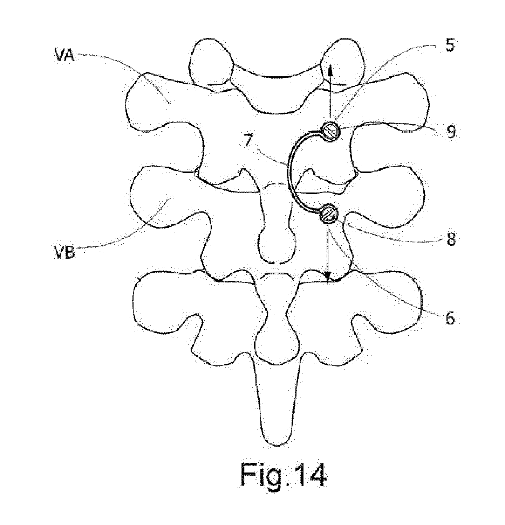

While reference has been made to the method and staple actuating with the distraction force over the end plates, growth plates or epiphysis of the vertebrae, the invention also provides the application of the staple into only one vertebrae or bone that must be corrected in its curvature or properly aligned. If one or more vertebrae must be deformed to correct a spine deformation, one or more devices of the invention can be affixed to one or more vertebrae, to promote growth of the bone at a desired pattern or to relieve the pressure. The staple can be applied more posterior if there is also a need to correct a lordotic (hypokyphosis) deviation, as it is found very frequently. The method and staple may be also applied to other disorders such as Kyphosis, that is the increase of the normally occurring posterior dorsal curvature of the spine, and Lordosis, which is the opposite deformity, the dorsal spine is abnormally straight or has a concavity where normally there is convexity. According to the specific deformity the staple will be connected to the vertebrae by means of a screw, for example by a posterior surgical approach as shown in Figure 14.

For the posterior surgical approach the rings are affixed by conventional screws to the pedicles of two neighbour vertebrae "Va", "Vb", reaching the vertebral body. The inventive device is implanted with the rings on the concave side of the curvature of the spine. When the nitinol reaches the transformation temperature, it will transmit a traction force to the screws and to the intervertebral space and the end plate, thus attempting to straighten the curve.

The kyphosis deformity can also be corrected by the present inventive method by applying a staple, on the left side of the vertebrae, through a posterior approach, to the pedicles of two neighbour vertebrae at the apex of the deformity and by applying another staple to the pedicles on the right side of the two vertebrae. This procedure can be used on two or more pair of vertebrae.

Alternative approaches or positions may be elected according to the needs, for instance, it is very frequently found that there is rotation component, besides the lateral curve, in all the scoliotic curves. In this situation, the staples may be applied, connected or inserted, obliquely regarding the plane of the intervertebral space. As the distraction force takes place, there will be also, a oblique force exerted on the intervertebral space, producing a rotating force between the neighbor vertebrae. Therefore, both deviations, namely lateral and rotation deviations, may be corrected at the same time.

Also according to the invention, the staple may have several shapes, for example, the inventive staple may define, a rectilinear rod, a curved rod, a "U" rod, and legs.

While only some preferred embodiments of the invention have been illustrated and disclosed, the same are examples which are not limitative or restrictive of the scope of protection. On the contrary, it must be clearly understood that many other embodiments, modifications and alterations equivalent to the elements of the invention may be suggested by persons skilled in the art after reading the present description, without departing from the spirit of the present invention and/or the scope of the appended claims.

## Claims

1. A device for correction of bone disorders **characterized by**:
an elongated wire entirely made of a shape-memory alloy, thermally treated to have at least one portion exhibiting a shape memory characteristic and at least one remaining portion without exhibiting said shape memory characteristic, and with the at least one remaining portion mechanically deformed during the thermal treating thereof to have a permanent desired shape.

2. The device of claim 1, **characterized in that** the elongated wire is entirely made of a material exhibiting a shape memory characteristic, with the at least one remaining portion defining two opposite ends of the wire and the at least one portion defining an intermediate portion extending between the two opposite ends wherein,
at least one of the two opposite ends is thermally treated in a manner to eliminate the shape memory characteristic of the elongated wire just in said at least one opposite end while keeping the shape memory effect in the intermediate portion of the wire.

3. The device of claim 2, **characterized in that** each of said two opposite ends is mechanically deformed into a ring for receiving a screw.

4. The device of claim 2, **characterized in that** the wire cross-section is selected from the group consisting of circular and quadrangular.

5. The device of claim 3, further **characterized by** a screw inserted in the ring, with the screw having a head with a rounded bottom pivotally retained into the ring.

6. The device of claim 3, further **characterized by** a screw inserted in the ring, with the screw having a washer and a nut and with the ring sandwiched between the washer and the nut in a manner that the device and the screw remain firmly retained at a relative position.

7. A device for correction of bone disorders **characterized by** a body made of a material exhibiting a shape memory characteristic, the body having a first portion thermally treated and mechanically deformed in a manner to have the shape memory characteristic eliminated and a desired shape, and a second portion exhibiting said shape memory characteristics.

8. The device of claim 7, **characterized in that** the body is an elongated wire with the first portion being straight and the second portion being a ring located at an end of the first portion.

9. The device of claim 7, **characterized in that** the body is an elongated wire with the first portion being straight and the second portion being a ring located at each one of two opposite ends of the first portion.

10. A method of making a device according to claim 1, **characterized by** the steps of:
providing a length of a wire made of a shape memory alloy;
defining at least one portion and at least one remaining portion of the wire;
forming the at least one portion of the wire into a desired shape;
thermally treating the at least one remaining portion of the wire in order to set the at least one remaining portion to exhibit a shape memory characteristic.

11. A method of making a device according to claim 2, **characterized by** the steps of:
providing a length of a wire made of a material exhibiting a shape memory characteristic,
defining the two opposite ends and the intermediate portion of the wire, with the intermediate portion extending between the two opposite ends,
thermally and mechanically treating at least one opposite end of the two opposite ends to eliminate the shape memory characteristic of the elongated wire just in said at least one opposite end and deform the elongated wire into a desired shape just in said at least one opposite end while keeping the shape memory effect in the intermediate portion of the wire.

12. The method of claim 11, **characterized in that** the step of thermally treating comprises heating the opposite ends at a temperature between about 600°C to well below 1300°C.

13. The method of claim 11, **characterized in that** the step of thermally treating at least one end of the two opposite ends comprises heating the at least one end preventing the intermediate portion from being heated, bringing the heated at least one end into a shape of a ring and letting the ring-shaped end cool down to room temperature.

14. The method of claim 11, **characterized in that** the step of thermally treating at least one end of the two opposite ends comprises inserting the at least one end into a furnace and heating the at least one end, preventing the intermediate portion from being heated, removing the at least one end from the furnace, bringing the heated at least one end into a shape of a ring and letting the ring-shaped end cool down to room temperature.

15. A method of making a device according to claim 7, **characterized by** the steps of:
providing a body made of a material exhibiting a shape memory characteristic,
defining a first portion and a second portion of the body,
thermally treating the first portion of the body to eliminate the shape memory characteristic of body while keeping the shape memory effect in the second portion of the body, and mechanically deforming, during the thermal treating, the first portion to bring it into a desired shape.
